# EUROPEAN PATENT SPECIFICATION

(11) **EP 2 306 919 B1**
(45) Date of publication and mention of the grant of the patent: **21.03.2012**
(21) Application number: 09740015.4
(22) Date of filing: 04.05.2009
(51) Int. Cl.: A61B 18/20

(54) **LASER ENERGY DEVICES FOR SOFT TISSUE REMOVAL**
LASERENERGIEVORRICHTUNGEN ZUR ENTFERNUNG VON WEICHTEILEN
DISPOSITIFS A ENERGIE LASER POUR L'ENLEVEMENT DE TISSU MOU

(30) Priority: 02.05.2008 US 49829 P
(43) Date of publication of application: 13.04.2011
(73) Proprietor: CURVE MEDICAL LLC, Minneapolis MN 55405 (US)
(72) Inventor: WOOLFSON, Steven, Boston, MA 02205 (US); DRESSEL, Thomas, Bloomington, MN 55438 (US); SOWYRDA, Paul, Needham, MA 02494 (US); TOBIN, Stephen, M., Newton Highlands, MA 02461 (US); ZELICKSON, Brian, D., Minneapolis, MN 55405 (US)
(74) Representative: Harrison IP Limited
(86) International application number: PCT/US2009/042727
(87) International publication number: WO 2009/135213

(56) References cited:
- EP-A- 0 821 916
- WO-A-95/24867
- WO-A-2008/073985
- GB-A- 2 219 213
- US-A- 4 985 027
- US-A- 5 102 410
- US-A- 5 380 316
- US-A1- 2004 068 256
- US-B1- 6 620 154
- US-B1- 6 669 685

## Description

### FIELD OF THE INVENTION

This invention relates to devices for improving the surgical procedure of soft tissue removal by lipolysis. This invention has immediate and direct application to the surgical procedure of liposuction or body contouring as well as application in the surgical procedures of other soft tissue removal such as brain tissue, eye tissue, and other soft tissue.

### BACKGROUND OF THE INVENTION

Within the past decade, the surgical use of lasers to cut, cauterize and ablate tissue has been developing rapidly. Advantages to the surgical use of laser energy lie in increased precision and maneuverability over conventional techniques. Additional benefits include prompt healing with less post-operative pain, bruising, and swelling. Lasers have become increasingly important, especially in the fields of ophthalmology, gynecology, plastic surgery and dermatology, as a less invasive, more effective surgical therapeutic modality which allows the reduction of the cost of procedures and patient recovery times due to diminished tissue trauma, bleeding, swelling and pain. The C02 laser has achieved wide spread use in surgery for cutting and vaporizing softtissue. The C02 laser energy has a very short depth of penetration, however, and does not effectively cauterize small blood vessels. Other means such as electrocautery must be used to control and minimize blood loss. Infrared lasers such as the Neodymium-doped yttrium aluminum garnet ("Nd:YAG") laser, e.g. a Nd:Y3Als012 laser, on the other hand, can effectively vaporize soft tissue and cauterize small blood vessels because of greater depth of tissue penetration. But the greater depth of tissue penetration introduces a risk of unwanted damage to deeper tissues in the path of the laser energy beam. Accordingly, infrared lasers have achieved limited use in the field of soft tissue surgery.

Recently, some infrared wavelengths have been shown to have selectivity to lipids and adipose tissue. The potential benefit of these wavelengths it that they can selectively melt or destroy fat with less energy while sparing other surrounding tissues such as nerves and collagen. In addition, various visible light lasers have shorter wavelengths and therefore do not penetrate deeply into tissue, while having the benefit of being able to selectively target structures such as blood vessels to help control bleeding.

Liposuction, a surgical technique of removing unwanted fat deposits for the purpose of body contouring, has achieved widespread use. In the U.S., over 400,000 liposuction procedures were performed in 2005 alone. The liposuction technique utilizes a hollow tube or cannula with a blunt tip and a side hole or tissue aspiration inlet port near its distal end. The proximal end of the cannula has a handle and a tissue outlet port connected to a vacuum aspiration pump. In use, a small incision is made in the patients skin near the tissue removal site. The cannula tip is inserted through the incision the tissue aspiration inlet port is passed beneath the surface of the skin into the unwanted fat deposit. The vacuum pump is activated, drawing a small amount of tissue into the lumen of the cannula via the aspiration inlet port. Longitudinal motion of the cannula removes the unwanted fat by a combination of sucking and ripping actions. The ripping action, while effective, can cause excessive trauma to the fatty tissue and surrounding tissue resulting in considerable blood loss and post-operative bruising, swelling, and pain. Proposed advances in the techniques and apparatus in this field have been primarily directed to the design of the aspiration cannula, and more recently have involved the application of ultrasound and irrigation to melt and solubilize fatty tissue or the use of an auger within the lumen of the cannula to facilitate soft tissue removal. These proposed advances do not adequately address the goals of the surgical procedure: the efficient and precise removal of soft tissue with minimal tissue trauma and blood loss.

Laser energy devices have been developed that are a modification of a suction lipectomy cannula. Such devices position soft tissue within a protective chamber, allowing an Nd:YAG laser energy beam to cut and cauterize the soft tissue within the chamber, without fear of unwanted damage to surrounding or deeper tissues. Thus, soft tissue can be removed without the ripping action inherent in the conventional liposuction method. Accordingly, tissue trauma can be reduced. Furthermore, the elimination of the ripping action of the conventional liposuction method expands the potential scope of soft tissue removal. However, the effectiveness and efficiency of existing laser energy devices may be limited, for example, by the interior positioning of the Nd:YAG laser fiber (i.e. by the running of the laser fiber through the cannula lumen). Such positioning can decrease the cross-sectional area of the lumen which can lead to clogging and decreased efficiency. Furthermore, in previous designs, the terminal end of the laser fiber is positioned proximal to the aspiration inlet port of the liposuction cannula. This can be disadvantageous because as the removed soft tissue is suctioned from the removal site, it is drawn directly into the firing end of the fiber causing charring and destruction of the laser fiber tip.

Further, existing devices may be limited to the use of a single wavelength Nd:YAG laser. Accordingly, such devices are not able to selectively target specific structures such as fat and blood vessels. In addition, it is necessary to enclose the fiber tip of such devices to minimize injury to surrounding vital structures.

Additionally laser energy devices can expand the surgical applicability of the liposuction method. Generally, the liposuction method is limited to the aspiration of fat. Other soft tissues, such as breast tissue, lymphangiomas, hemangiomas, and brain tissue are too dense, too vascular, or too precariously situated to allow efficient and safe removal utilizing the liposuction method. The laser energy devices utilize a precise cutting and coagulating action of the laser within the cannula, thereby permitting the removal of these dense or vascular soft tissues. This laser can be used, for example, in the precise removal of brain tissue without fear of unwanted damage to surrounding or deeper tissues. Furthermore, the C02 laser is extensively used for the vaporization of brain tumors, but because of its inability to effectively coagulate blood vessels, other methods such as electrocautery must be used to control blood loss during the procedure. In addition, because the vaporization of tissue generates large volumes of noxious and potentially toxic smoke, expensive, noisy and cumbersome suction devices must be used to eliminate the smoke from the surgical field. However, laser energy devices utilizing the more effective coagulating power of visible and infrared lasers permit the combined action of tissue cutting, control of blood loss, and elimination of smoke from the surgical field.
US 4,985,027 and US 5,102,410 both disclose a laser soft tissue aspiration device comprising an aspiration cannula housing a laser energy transmitting means for conducting laser energy to the site within a patient's body for aspiration of soft tissue. The cannula is provided with an aspiration inlet port adjacent the cannula distal end. The proximal end of the cannula is provided with fluid flow connection to an aspiration source. A laser guide tube is additionally provided housing the laser energy transmitting means extending longitudinally within the cannula lumen from the laser energy source at the cannula proximal end and terminating at a point immediately prior to the aspiration inlet port. The tube also provides a conduit for transmitting cooling and cleaning fluid flow for the laser energy transmitting means.
EP 0 821 916 A2 discloses A method for the photodisruption of tissue using a laser beam for stereotactic laser neurosurgery includes the initial step of positioning the distal end of a probe into the tissue to be photodisrupted. A laser beam having laser pulses of picosecond or femto-second duration is then directed along a beam path through the probe. Using a focusing lens, which is slidably positioned on the beam path in the probe, and a light reflector, which is slidably positioned on the beam path at the distal end of the probe, the laser beam is focused to a focal point that is located on a line substantially perpendicular to the beam path through the probe. By concerted movement of the lens and mirror, predetermined cylindrical layers of tissue can be photodisrupted. Specifically, a rotation of the mirror causes photodisruption of tissue along a circular arc and a simultaneous movement of the lens and mirror allows for photodisruption of additional arcs on a cylindrical surface. Further, by moving the lens relative to the mirror, cylindrical tissue surfaces having different radii can be photodisrupted. Additionally, the area of tissue photodisruption can be irrigated and aspirated during practice of the method.
The US 2004/068256 A1 discloses an electromagnetically induced cutting mechanism provides accurate cutting operations on soft tissues. The electromagnetically induced cutter is adapted to interact with atomized fluid particles. A tissue remover comprises an aspiration cannula housing a fluid and energy guide for conducting electromagnetically induced cutting forces to the site within a patient's body for aspiration of soft tissue. The cannula is provided with a cannula distal end. The proximal end of the cannula is provided with fluid flow connection to an aspiration source. Separated soft tissue and fluid are aspirated through the cannula distal end and the cannula by an aspiration source at the proximal end of the cannula.

### SUMMARY

Embodiments of the invention include devices for performing soft tissue removal by lipolysis. Devices according to some embodiments comprise an aspiration cannula which can be inserted to a tissue removal site within a patient. The device can deliver laser energy to the tissue removal site for ablating targeted tissue. Ablated tissue can then be removed from the site by the aspiration cannula.

In one aspect, the invention features an optical delivery system for a laser soft tissue aspiration device. The optical delivery system includes a laser energy transmission guide, a lens, and a reflective surface. The laser energy transmission guide can include a terminal point, from which laser energy can be delivered. The laser energy can be reflected across an aspiration inlet port of the laser soft tissue aspiration device by the reflective surface. A lens can be in optical communication with the laser energy transmission guide and adapted to isolate the terminal point from a lumen of the laser soft tissue aspiration device. The reflected laser energy can ablate soft tissue suctioned into the laser soft tissue aspiration device. In some embodiments, the optical delivery system can be contained within a tip assembly, sized to fit over an open cannula end of the laser soft tissue aspiration device. In such an embodiment, the lens and the reflective surface can be installed within the tip assembly. Alternatively, the optical delivery system can be contained within a cannula of the laser soft tissue aspiration device.

In another aspect, the invention features a laser soft tissue aspiration device. The laser soft tissue aspiration device includes an aspiration cannula, a laser energy transmission guide, and an optical delivery system. The aspiration cannula has a proximal end and a distal end and defines a lumen which is provided with fluid flow connection to an aspirated soft tissue outlet port at the cannula proximal end. In addition, at least one aspiration inlet port is formed within the aspiration cannula proximate the distal end and in fluid flow connection to the lumen. The laser energy transmission guide can extend from a laser energy source at the aspiration cannula proximal end to a terminal point proximate the cannula distal end. The laser energy transmission guide is configured to transmit laser energy from the laser energy source and to the terminal point. The optical delivery system is disposed at the distal end of the cannula and is in optical communication with the laser energy transmission guide. The optical delivery system can include a lens and a reflective surface. The lens can be disposed distally relative to the aspiration inlet port and adapted to isolate the terminal point from the cannula lumen. The reflective surface can be disposed distally relative to the lens, and adapted to direct laser energy across the aspiration inlet port. In some embodiments, the optical delivery system can be installed within a tip assembly sized to fit over an open cannula end of the laser soft tissue aspiration device. Alternatively, in some embodiments, the optical delivery system can be installed within the distal end of the aspiration cannula lumen.

In another aspect, the invention features a soft tissue aspiration device. The soft tissue aspiration device includes a hand-manipulatable, elongate cannula having proximal and distal ends. The cannula defines a lumen which is provided with fluid flow connection to an aspirated soft tissue outlet port at the proximal end of the cannula. At least one aspiration inlet port can be provided proximate the cannula distal end and in fluid flow connection to the lumen. A laser energy transmission guide can deliver laser energy from a laser energy source to a terminal point at the cannula distal end. To protect the laser energy transmission guide, the terminal point of the laser energy transmission guide can be positioned distally relative to the proximal end of the aspiration inlet port and configured to direct laser energy within the lumen.

In another aspect, the invention features another soft tissue aspiration device. The soft tissue aspiration device includes a cannula having a proximal end and a distal end. The cannula defines an aspiration lumen provided with fluid flow connection to a suction source at the proximal end. At least one aspiration inlet port can be provided within the cannula distal end in fluid flow connection to the aspiration lumen. The device can further include a laser energy transmission guide adapted to deliver laser energy from a laser energy source to a terminal point at the cannula distal end. The terminal point of the laser energy transmission guide can be isolated.

According to yet another aspect, the invention includes a device for delivering laser energy to a lipolysis site within a patient. The laser energy can be used to ablate targeted tissue. The device can include a rigid laser energy transmission guide having a proximal end, a distal end, and a working distal tip at the distal end. The working distal tip can be adapted to deliver laser energy to the lipolysis site. A junction can be included at the proximal end. The junction can provide the rigid laser energy transmission guide an optical connection to a laser energy source.

These and various other features and advantages will be apparent from a reading of the following detailed description.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.
Figure 1 is a side cut-away elevation view of a soft tissue aspiration device known in the art.
Figure 1A is a partial exploded longitudinal section of a laser energy transmission guide known in the art.
Figure IB is a partial exploded longitudinal section of a laser guide tube known in the art.
Figure 2 is a side cut-away elevation view of a tip assembly disposed about the distal end of a cannula according to one embodiment of the first aspect of the invention.
Figure 3 is a side cut-away elevation view of a tip assembly disposed about the distal end of a cannula according to another embodiment of the first aspect of the invention.
Figure 4 is an optical schematic of the embodiment of Figure 2.
Figure 5 is a side cut-away elevation view of the distal end of a cannula having an optical delivery system installed according to one embodiment of the first aspect of the invention.
Figure 6 is a side cut-away elevation view of the distal end of a cannula having an optical delivery system installed according to another embodiment of the first aspect of the invention.
Figure 7 is an optical schematic of the embodiment of Figure 5.
Figure 8 is a perspective view of an embodiment of laser soft tissue removal device according to a second aspect of the invention.
Figure 9 is a side cut-away view of the embodiment of Figure 8.
Figure 10 is a perspective view of another embodiment of a laser soft tissue removal device according to a second aspect of the invention.
Figure 11 is a perspective view of an embodiment of a rigid laser energy transmission guide according to a second aspect of the invention.

### DETAILED DESCRIPTION

The following detailed description is exemplary in nature and is not intended to limit the scope, applicability, or configuration of the invention in any way. Rather, the following description provides practical illustrations for implementing exemplary embodiments of the present invention. Those skilled in the art will recognize that many of the examples provided have suitable alternatives that can be utilized.

The embodiments of the present invention described below are not intended to be exhaustive or to limit the invention to the precise forms disclosed in the following detailed description. Rather, the embodiments are chosen and described so that others skilled in the art can appreciate and understand the components, principles and practices oft he present invention.

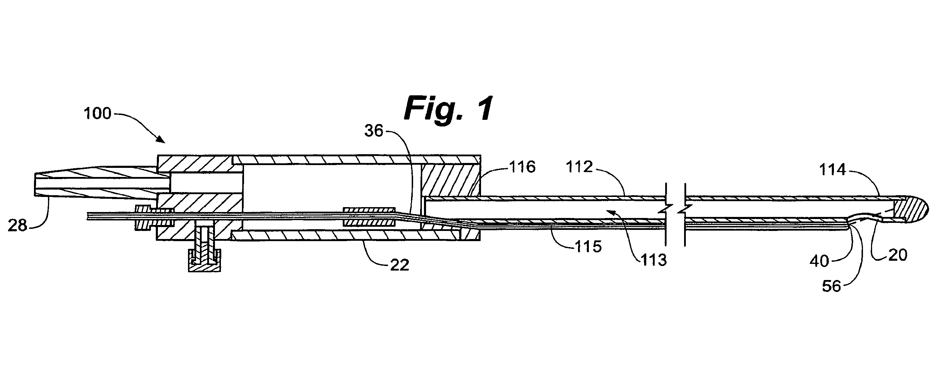

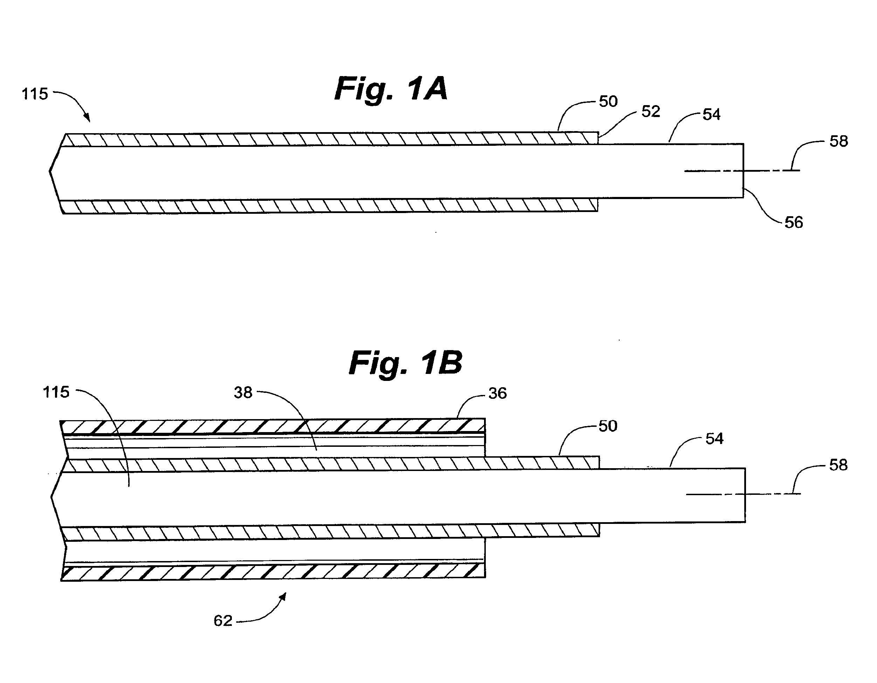

In a first aspect, an improved aspiration cannula tip for delivery of laser energy in laser soft tissue aspiration devices is provided. As a reference, Figure 1 depicts an exemplary prior art laser soft tissue aspiration device 100 wherein the device comprises an aspiration cannula 112, a laser guide tube 36, an aspiration inlet port 20, and a laser energy transmission guide 115. The aspiration cannula 112 includes a lumen 113 providing for fluid and/or soft tissue flow within the cannula 112. The lumen 113 is in communication with one or more aspiration inlet ports 20 at a distal end 114 of the aspiration cannula 112. An aspirated soft tissue outlet port 28 at a proximal end 116 of the device 100 and in fluid flow connection to the lumen 113 can couple an aspiration source (not shown) with the lumen 113. The aspiration source can comprise generally any suction source such as, for example, a vacuum pump aspiration source or syringe plunger suction source. The device also includes a laser guide tube 36 extending longitudinally along the 112 to a termination point 40 proximal the aspiration inlet port(s) 20. A laser energy transmission guide 115 extends within the laser guide tube 36 from a laser energy source (not shown) to the termination point 40 at the distal end 114 of the cannula 112. A handle 22 is included at the proximal end 116 of the aspiration cannula 112. In this particular embodiment, the laser guide tube 36 and laser energy transmission guide 115 traverse the cannula 112 length exterior to the lumen 113. However, as will be made clear below, embodiments of the invention can be adapted for use with other cannula and laser guide tube/laser energy transmission guide arrangements. Moreover, like the laser soft tissue aspiration device 100 of Figure 1, embodiments of the invention result in orientations of the distal end 56 of the laser energy transmission guide 115 that direct laser energy across the face of the aspiration inlet port(s) 20 such that the laser energy remains generally within the lumen 113.

An exemplary laser energy transmission guide 115 can be seen in Figure 1A. Such a guide can include a laser fiber sheath 50 encasing laser fiber 54. The sheath 50 and fiber 54 are generally coaxial about longitudinal axis 58, with the fiber 54 protruding from the sheath 50 at sheath termination point 52 and laser energy emanating from fiber end 56. The portion of fiber protruding from the sheath will later be referred to as the fiber tip. In various embodiments of the present invention, the laser fiber sheath 50 is a Teflon laser fiber sheath. Suitable laser fiber 54 materials can include: synthetic laser fibers, glass, quartz, sapphire or other optically transmissible materials.

An exemplary laser guide tube 36 is shown in Figure 18. In this embodiment, the laser guide tube 36 generally includes an outer tube defining a laser guide lumen 38. A laser energy transmission guide 115 can be disposed within the laser guide lumen 38. In this embodiment, the laser energy transmission guide 115 comprises the laser energy transmission guide of Figure 1A. Further, in some embodiments, the laser guide lumen 38 can be filled with a filler material, such as an epoxy, along the length of the laser guide tube 36. Such a filler material can affix the laser energy transmission guide 115 within the laser guide tube 36. Moreover, a filler material can
act as a heat-sink. In some embodiments, filler material can include metal or conductive fragments (e.g. aluminum, copper, etc.) dispersed throughout to increase the thermal conductivity of the filler material 132 and better draw heat away from the laser energy transmission guide 115 to prevent charring of the fiber. Alternatively, the laser guide tube 36 can be of sufficient internal diameter to accommodate a fluid and laser fiber guide tube system. For example, the laser guide tube can accommodate a fluid and laser fiber guide tube system such as that described in U.S. Pat. Pub. No. 2008/0208105. When used with such systems, the laser guide lumen 38 can act as a coaxial fluid channel to provide for fluid cooling of the laser energy transmission guide 115 along its length.

In addition, some embodiments can include a sensor within the device adapted to control the application of laser energy through the device. For example, some embodiments can include a temperature sensor, which prevents the device from delivering laser energy when the temperature at the tissue removal site, or within the device exceeds a prescribed threshold. Other sensors can likewise be utilized, for example a suction sensor may be provided within the cannula. Such a sensor can be used to indicate whether suction is being properly provided throughout the cannula lumen. In the event of a clog or occlusion of the cannula lumen, the sensor can trigger an alarm, e.g. a visual or audible alarm, to let the practioner know that suction is no longer being provided to the tissue removal site. Alternatively, in the event of a clog or occlusion, the sensor may be able to terminate the operation of the device. Further, some embodiments can include a motion sensor. Such a sensor can be configured to determine whether the cannula is being moved. This information can be used, for example, to allow for laser energy to be delivered only while the cannula is moving longitudinally or otherwise within the patient.

In use, an operator makes short incision in the patient's skin near the site of tissue removal and the cannula 112 is passed into the soft tissue to be removed. The aspiration pump is activated, generating negative pressure within the lumen 113, thereby drawing soft tissue through the aspiration inlet port 20. The laser source is then activated, causing laser energy to be transmitted to the terminal point of the laser fiber 56 and into the soft tissue within the cannula lumen 113, cleaving the soft tissue and coagulating small blood vessels. Additional soft tissue enters the soft tissue inlet port 20 by virtue of a reciprocating longitudinal motion of the laser soft tissue aspiration device 100 within the soft tissue. The suction within the device then draws the aspirated soft tissue through the soft tissue outlet port 28, where it is disposed of. It should be noted that the above described use, is merely an exemplary use of the prior art device of Figure 1, and should not be construed so as to limit use of embodiments of the invention.

In a first aspect, embodiments of the invention include an optical delivery system comprising at least a lens and a reflective surface adapted for use with laser soft tissue removal devices such as those discussed above. The optical delivery system isolates the tip of the laser energy transmission guide from the cannula lumen, thereby preventing occlusion and build up of ablated soft tissue near the laser energy delivery tip. Thus, laser energy can be delivered more consistently about the aspiration inlet port. Moreover, the lens is configured to direct laser energy in a desired manner to the lumen allowing for collimating or converging of laser energy.

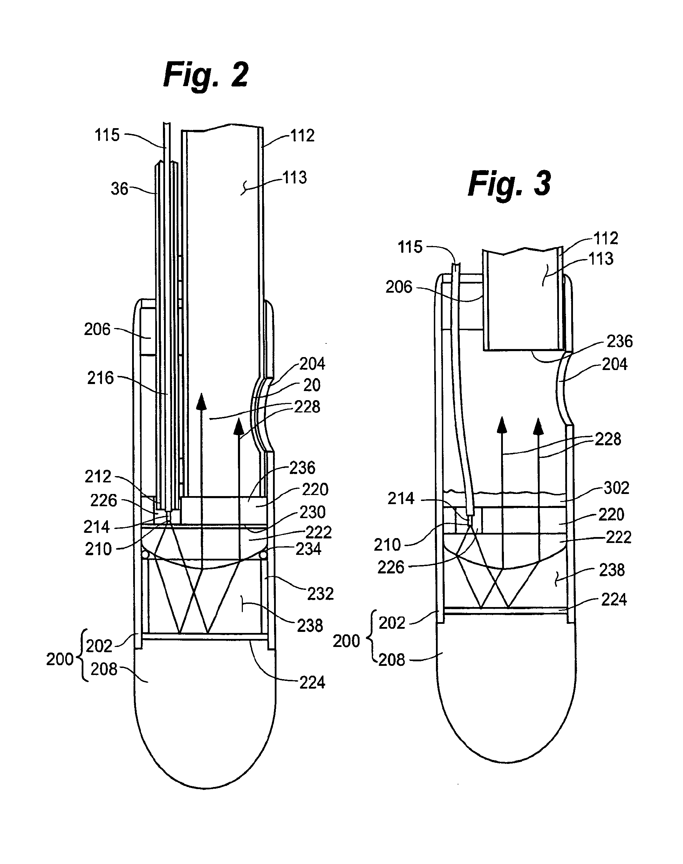

In some embodiments, the optical delivery system can be included within a tip assembly. For example Figures 2 and 3 show embodiments including a tip assembly 200 mated with the distal end of a cannula 112. Such embodiments include an outer tube 202 that can be disposed about the distal end of the cannula 112. In some embodiments, the outer tube 202 can include one or more tip ports 204 to provide an inlet to the cannula lumen 113. Tip ports 204 can be arranged to align with aspiration inlet ports 20 on the cannula 112 (see e.g. Figure 2), or in other embodiments, the tip port 204 can be positioned distally relative to an open-ended cannula 236 (see e.g. Figure 3). An adhesive 206, for example epoxy, or other means may be used to secure the tip assembly 200 to the cannula end. Further, a tip 208 can be installed about the distal end of the outer tube 202. The tip 208 can be a disposable tip, removably connected (e.g. by threaded-, snap-, pin-, or other connection) to the outer tube 202. Or, a separate tip can be fixedly connected (e.g. by adhesive, weld, or other connection) to the outer tube 202. Alternatively, in some embodiments, the tip 208 is not separate from the tube 202, but is formed out of the tube 202, i.e. the distal tube end can be sealed and machined to a rounded, bullet or otherwise shaped end. In some embodiments, the tip assembly 200 is approximately 5 cm in length.

Figure 2 shows an embodiment including a tip assembly 200 adapted to be fit about a cannula having a laser guide tube 36 running external to the cannula 112. Here, laser energy transmission guide 115 extends within the laser guide tube 36 which has been fixedly coupled (e.g., by weld) external to the cannula 112. Laser energy transmission guide 115 terminates at a terminal point 210 proximate the distal end 210 of the laser guide tube 36. As seen in this embodiment, laser energy transmission guide 115 can include a fiber tip 214, protruding from a sheath 216. In some embodiments, the laser energy transmission guide can be fixed into place, e.g. by epoxy bond, within the laser guide tube. In other embodiments, the laser energy transmission guide 115 is free within the laser guide tube 36. Such an arrangement may be useful where the laser energy transmission guide 115 is coupled to, or provided with a laser energy source (not shown). In this case, the cannula 112 can be provided separately from the laser energy source, and the laser energy transmission guide of the source can be threaded from a handle or other proximal end access, through the laser guide tube 36 to the terminal point 210. In embodiments having a stainless steel laser guide tube, terminal point 210 is preferably disposed at or beyond the open distal end 212 of the laser guide tube 36 as shown in Figure 2. Such an arrangement can minimize the laser energy that would be dissipated were the stainless steel guide tube to be used as a wave guide. However, in some embodiments, the laser guide tube 36 can be used as a wave guide, i.e. terminal point 210 can be positioned proximally within the laser guide tube 36.

In this embodiment, the optical delivery system includes a window 220, a lens 222, and a reflective surface 224 disposed within the outer tube 202. The window 220 spans an interior circumference of the outer tube 202 and is positioned proximally relative to lens 222 yet distally relative to the cannula lumen 113 and aspiration inlet ports 20. Window 220 comprises a rigid, optically transmissive material such as glass or plastic. In a preferred embodiment, the window comprises Borosilicate glass or fused quartz. In some embodiments, window 220 can include a hole 226 adapted to receive the laser guide tube 36 and/or laser energy transmission guide 115 when the window 220 is abutted against the distal end of the cannula 112. For example, in Figure 2, a portion of laser guide tube 36 extends distally beyond the end of cannula 112 and is received by hole 226 in the window 220. Such an arrangement can be used to optimally position the tip assembly 200 about the cannula 112. The window 220 and/or lens 222 can be used to isolate the aspiration cannula lumen 113 from the laser delivery components, namely the laser energy transmission guide 115, fiber tip 214, and lens 222.

The optical delivery system further includes a lens 222 adjacent to window 220. In operation, lens 222 directs laser energy 228 emitted by the laser energy transmission guide 115 across the aspiration inlet port 20. Lens 222 may further be used to focus, collimate, or diffuse laser energy within the lumen 113 so that effective tissue ablation may be accomplished. The material, refractive index, and shape of the lens can depend on the characteristics of the laser energy to be delivered. For example, in many lipolysis applications, it is desirable to deliver from 7-25 Watts of laser energy having a wavelength of 800 - 1000 nm, to target area having a size of approximately 2-20 mm². In a preferred embodiment, the lens is concave and made of BK-7 crown glass having a refractive index of approximately 1.5. Due to differences in refractive index, the junction 230 between the window 220 and lens 222 can be a source of Fresnel reflection loss, i.e. loss of energy due to light energy being reflected back toward the source at the interface between the media. To avoid or decrease this loss, and therefore increase laser performance, the junction 230 may include an index matching substance, e.g. a gel or an adhesive. An index matching substance should be selected to minimize the step change in the refractive index between the window and lens.

In many embodiments, the optical delivery system uses a reflective surface 224 to direct laser energy across the aspiration inlet ports 20. The reflective surface 224 may comprise a mirror, polished metal (e.g. copper), a "hot" mirror (e.g. a hard layer stack including dielectric and/or reflective materials deposited on an optical material such as glass) or other surface suitable for reflecting laser energy. The reflective surface is preferably a highly reflective metal in the wavelength range of 800-1100 nm. In some embodiments, it can be difficult and expensive to manufacture solid metallic mirrors. Moreover, some metallic mirrors can have energy loss on the order of, e.g., 5 % - 10 %. This lost light energy can be transformed into heat at the tip. Accordingly, some embodimetns comprise a hot mirror capable of reflecting the near-IR wavelengths, e.g. approximately 800 nm to 1,200 nm, and passing shorter wavelengths, e.g. below approximately 800 nm down to say approximately 400 nm. The shorter wavelengths passed by this mirror are not as easily absorbed by the metallic tip, and the longer wavelengths are reflected with a higher efficiency than a metallic mirror (1% loss typically). When used with a highly coherent laser beam at, for example, 850 nm +/- 50 nm, the shorter wavelengths are not present. Such mirrors can be made by depositing multiple layers of particular dielectric materials (e.g. zinc oxide, titanium oxide, tin oxide, silicon nitride...) and/or reflective materials (e.g. silver, gold, aluminum...) in a particular order onto a glass substrate.

In Figure 2, the reflective surface 224 is positioned adjacent to the tip 208 and is distally located relative to the lens 222. In operation, the reflective surface 224 reflects laser energy 228 delivered from the laser energy transmission guide 115 proximally within the lumen 113 and across aspiration inlet port 20 to cause ablation of soft tissue suctionally drawn into the lumen 113. A spacer 232 and o-ring 234 may be arranged within tip assembly 200 to retain lens 222 in a predetermined position relative to reflective surface 224. Spacer 232 can be a rigid cylindrical segment, made of the same material as the cannula, for example. O-ring 234 should be a generally resilient material, such as rubber, to provide some cushioning of the lens 222 against the spacer 232. When tip assembly 200 is installed about the cannula distal end, the lens 222 and window 220 can be compressed between the cannula distal end 236 and the spacer 232 and o-ring 234. Alternatively, in some embodiments, the lens 222 and window 220 can be affixed in position within the tip assembly by other means, such as for example adhesive. Thus in some embodiments, the lens 222 and reflective surface 224 are separated by a predetermined distance, providing tip space 238 between the two. This tip space 238 can be empty, or filled with an index matching gas, gel, or other substance. Alternatively, in some embodiments, the reflective surface 224 can be positioned so as to abut the lens 222 such that there is no separation between the two. Ultimately, refractive and physical characteristics of the lens 222, window 220, and tip space medium 238, reflective and physical characteristics of the reflective surface 224, and the distance between the components of the optical delivery system affect the dispersion of laser energy 228 within the lumen.

One of ordinary skill in the art will appreciate that additional optical delivery systems can be utilized according to the present invention. For example, an optical delivery system can comprise two or more reflective surfaces, or a shaped reflective surface that can redirect the laser beam multiple times, rather than a lens and a single reflective surface as described above. In such embodiments the laser beam is redirected by multiple reflective surfaces.

Figure 3 shows another embodiment including a tip assembly 200 comprising an outer tube 202 and tip 208. This embodiment is shown installed about a cannula 112 having an open distal end 236 and no aspiration inlet port. A tip port 204 within the outer tube 202 thus provides aspiration inlet to the lumen 113 via the open distal end 236. Moreover, this cannula design includes only an external laser energy transmission guide 115 without a laser guide tube. Of course, this embodiment can also be used with other cannula arrangements, for example, a cannula having an internal laser energy transmission guide or a laser guide tube such as that of Figure 2.

In this embodiment, the optical delivery system includes a window 220, lens 222, and reflective surface 224. Laser energy transmission guide 115 has been guided within the tip assembly 200, such that the terminal point 210 is within a hole 226 positioned within the window 220. As above, hole 226 is located to optimally position the fiber tip 214 within the optical delivery system. Optical characteristics of the embodiment are determined by the considerations discussed above. In other embodiments, not illustrated, the hole 226 may be positioned within the lens 222 to optimally position the fiber tip 214 within the optical delivery system and further protect the tip 214. In such embodiments, the optical delivery system may include or exclude the window 220.

In this embodiment window 220, lens 222, and laser energy transmission guide 115 are held in position by an epoxy layer 302 disposed proximally relative to the window 220. This epoxy layer 302 can comprise an optical epoxy, having optical characteristics allowing for transmission of laser energy 228 of desired wavelength. In some embodiments, the epoxy comprises EPO-TEK^{®} 353ND available from Epoxy Technology, Inc. 14 Fortune Dr., Billerica, MA 01821. In other embodiments, Norland No. 61 Optical Adhesive can be used. Application of the epoxy layer 302 about the proximal surface of the window 220 and circumferentially between the outer tube 202 and optical components can fix the window 220 and lens 222 in position. Moreover, the epoxy layer 115 can anchor the laser energy transmission guide 115 in position within the hole 226 of the window so that it is not displaced during use.

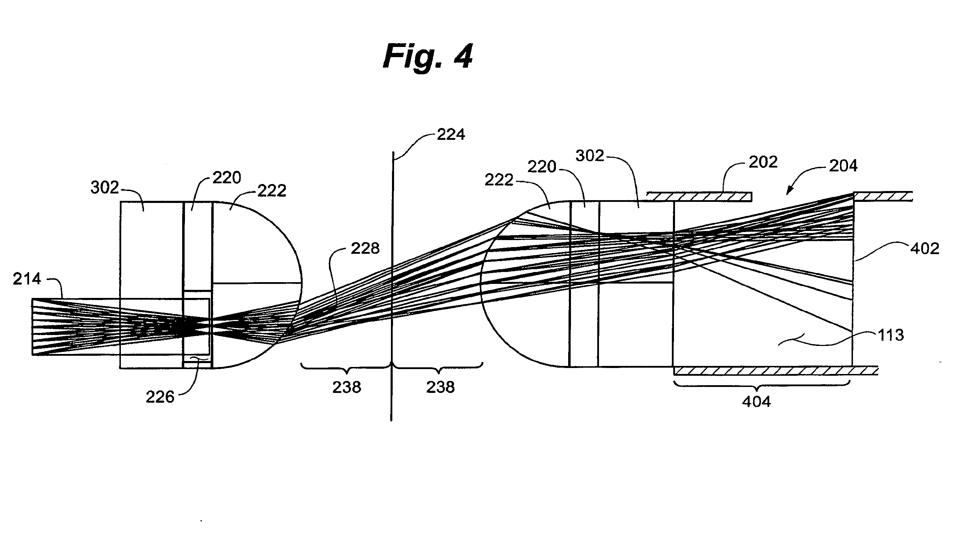

Figure 4 shows an unfolded optical schematic of a laser energy distribution pattern for an optical delivery system similar to that of Figure 3. In the schematic, the fiber tip 214 is shown abutting the lens 222. Rays of laser energy 228 dispersed from the fiber tip 214, pass through lens 222 and tip space 238 to reflect off of reflective surface 224 (depicted as passing through reflective surface in the unfolded view). The reflected rays again pass through tip space 238 and re-enter the lens 222, passing across junction 230, through window 220 and epoxy layer 302 before terminating at image plane 402. Image plane 402 represents a plane generally perpendicular to the proximal end of tip port 204 of the outer tube 202. Proximate the image plane 402, laser energy 228 would impact and ablate soft tissue suctioned through the port 204 and residing in the air/tissue space 404 between the image plane 402 and epoxy layer 302. Ablated soft tissue can then be aspirated through the cannula lumen 113. By the optical schematic of Figure 4, it is apparent that nearly all laser energy 228 is contained within lumen 113. To further ensure that laser energy is contained within lumen 113, embodiments of the invention may have a port 204 more distally located, thereby effectively moving image plane 402 distally toward lens 222. Alternatively, round or oval aspiration inlet ports can be radially offset on the cannula circumference. That is, rather than positioning the aspiration inlet port in the cannula 180 degrees circumferentially from the fiber tip, the port can be rotated to be, for example, 150 degrees from the fiber tip.

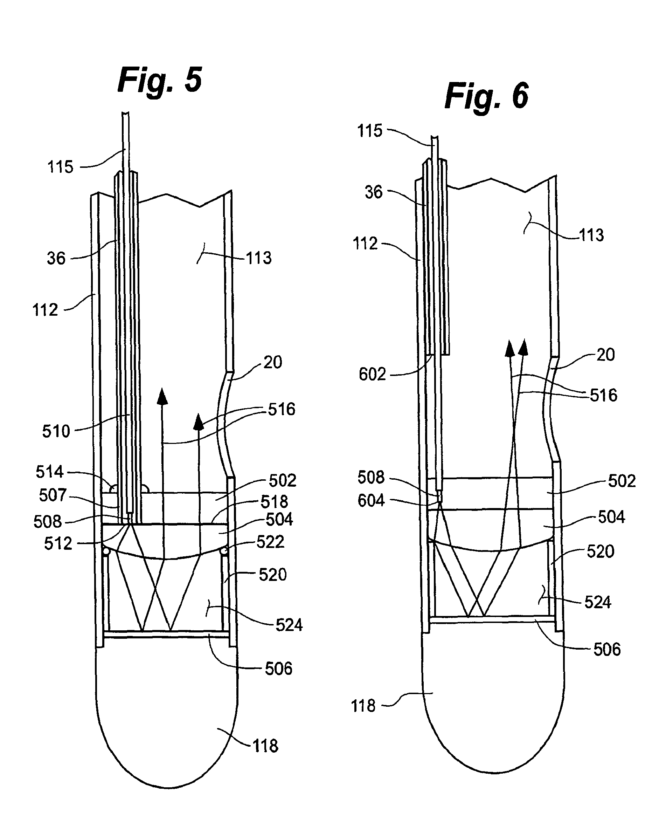

In some embodiments, for example those of Figures 5 and 6, the optical delivery system can be disposed within the distal end of a cannula. Such embodiments generally include a cannula 112 defining a lumen 113 and having at least one aspiration inlet port 20 proximate a distal end. The cannula distal end can be sealed and formed to a rounded, bullet, or otherwise shaped tip. Alternatively, a separate tip 118 can be installed about the distal end of the cannula 112. A separate tip 118 can be a disposable tip, removably connected (e.g. by threaded , snap , pin , friction fit, or other connection) to the cannula 112. In some embodiments, a separate tip 118 can be fixedly connected (e.g. by adhesive, weld, or other connection) to the cannula 112.

The optical delivery system of Figure 5 includes a window 502 and lens 504 disposed about an internal circumference of the cannula 112, and a reflective surface 506 distally located relative to the lens 504. Window 502 can be adapted to include a hole for receiving the distal end 507 of an internal laser guide tube 36 having a laser energy transmission guide 115 within. In this embodiment, the laser energy transmission guide 115 includes a fiber tip 508 protruding from a sheath 510 to a terminal point 512 located at the distal end of the laser guide tube 507. The distal end of the laser guide tube 507 is capped and sealed by the window 502 and lens 504, thereby physically isolating the fiber tip 508 from the lumen 113. In this embodiment, an epoxy bead 514 is applied at the joint between the laser guide tube 36 and window 502 to seal the connection and prevent the laser guide tube 536 from disengaging from the hole. In other embodiments, an epoxy layer (such as that in Figure 3) may be applied across the entire window surface to secure laser guide tube 36 within the window 502 and also to secure the window 502 within the circumference of the cannula 112. As above, the hole can be located in the window 502 to locate the fiber terminal point 512 to provide optimal dispersion of laser energy 516.

Lens 504 abuts both the window 502 and laser guide tube 36 at a junction 518. As described above, junction 518 may include an index matching gel for reducing Fresnel reflection across the junction. The lens 504 and window 502 can be secured within the cannula 112 by any means, for example adhesive, mechanical fastener, or frictional fitting. Preferably, the lens 504 remains in a fixed orientation relative to the aspiration inlet port 20 and reflective surface 506. A spacer 520 and o-ring 522, as described above, can be positioned between the lens 504 and tip 118 to provide appropriate tip space 524 to achieve the desired optical geometry.

Reflective surface 506 can be installed about a circumference of the cannula 112 distally located relative to the lens 504. In the embodiment of Figure 5, the reflective surface 506 is a generally flat mirror disposed across the proximal end of the tip 118.

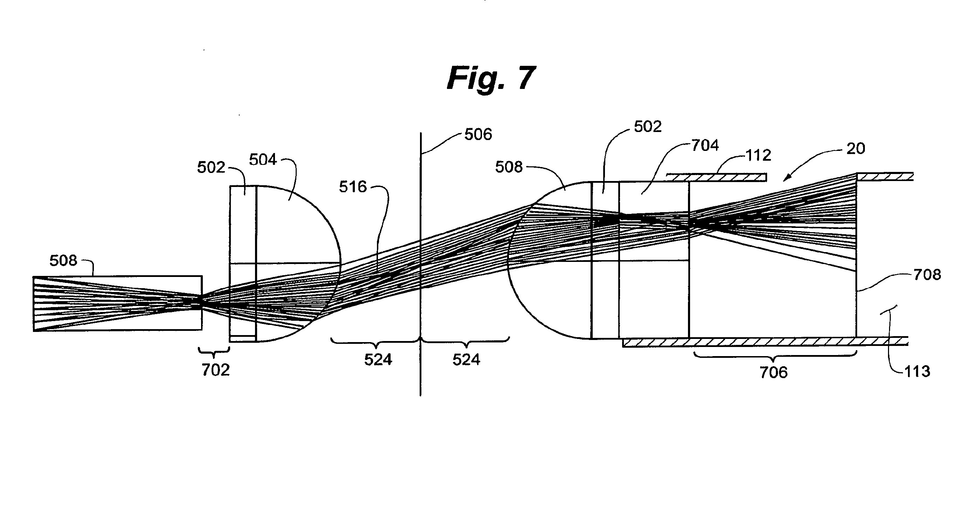

Figure 7 shows an optical schematic of an embodiment similar to that of Figure 5. In this embodiment, a fiber tip 508 has been disposed such that an air gap 702 exists between the fiber distal end and a window 502. In some embodiments, this air gap 702 can be approximately 1 millimeter. In calculating this optical schematic, the window 502 and lens 504 were constructed of silica, reflective surface 506 comprises a mirror, and a polycarbonate epoxy layer 704 (similar to epoxy layer 302 of Figure 3) was positioned proximally relative to window 502. An air tissue space 706 (e.g. of approximately 4-12 millimeters, in some embodiments 6 millimeters) can reside between the epoxy layer 704 and image plane 708, which is positioned at the proximal end of an aspiration inlet port 20. In this schematic, the image plane 708 shows an improved (i.e., less dispersed) distribution of laser energy 516 compared with the distribution of Figure 4.

The embodiment of Figure 6 is similar to that of Figure 5 in that the optical delivery system is disposed within the distal end of the cannula 112 and not a separate tube assembly. In this embodiment, several alternative features are illustrated. First, the cannula design includes an internal laser guide tube 36 having a laser guide tube terminal point 602 that is not sealed off by the optical delivery system as it has been in other embodiments. Such an arrangement can be particularly useful when the cannula 112 is adapted for use with a fluid and laser fiber guide tube system as described above. Because the laser guide tube 36 is not sealed off, a fluid can be delivered through laser guide tube 36 to cool laser energy transmission guide 115. Cooling fluid delivered through the guide tube can exit the tube at the laser guide tube terminal point 602 and be aspirated via lumen 113 along with removed soft tissue. Moreover, use of a cooling fluid may assist in the lipolysis process by helping to wash away removed soft tissue, thereby reducing the likelihood of occlusion of the lumen 113.

While the laser guide tube 36 of the embodiment of Figure 6 is in fluid communication with the lumen 113, the terminal point 604 of the laser energy transmission guide 115 can be embedded within window 502. Thus, fiber tip 508 (i.e., the distal end of the laser energy transmission guide) can remain isolated from the lumen 113 and aspirated soft tissue, thereby reducing the likelihood of charring of the tip 508. In some embodiments, the window 502 can be molded or otherwise formed about the distal end of the laser energy transmission guide 115. Other embodiments may include a window having a hole as above, with an epoxy bead or layer, or heat fused glass for sealing the fiber tip within the window. In embodiments that do not include a window, or include a combined window and lens structure, the fiber tip can be received by the lens in similar fashion. In some embodiments, a length of silicone tubing or other resilient material can be fit around the distal end of the fiber 508 as a sheath or sleeve, such that the fiber and silicone sleeve can provide a friction fit within the hole. Embodiments including a silicone or other resilient sleeve can provide for a protective seal of the fiber end, while allowing for lower cost fabrication and material requirements than other methods of sealing. In addition, such embodiments can be autoclaved, allowing for the cannula to be used to perform multiple procedures.

Other components of the optical delivery system of Figure 6 such as the lens 504, tip space 524, optional spacer 520 and o-ring, and reflective surface 506 are shown and can be analogous to those elements described above.

Although the above described embodiments have shown the use of a tip assembly only with cannulas having an external laser energy transmission guide (see e.g., Figures 2 and 3) and internal optical delivery systems only with cannulas having an internal laser energy transmission guide (see e.g., Figures 5 and 6), one should appreciate that other combinations and arrangements are possible. For example, a tip assembly can be adapted to fit about a cannula having an internal laser guide tube and laser energy transmission guide. Alternatively, a cannula having an external laser energy transmission guide can be adapted to include an internal optical delivery system. An internal laser energy transmission guide, should be understood to include devices in which the laser energy transmission guide runs from the proximal end of the cannula to the distal end of the cannula within the lumen of the cannula. In contrast, an external laser energy transmission guide is positioned outside of the cannula lumen.

Embodiments according to the present invention may further provide for protection of the laser energy transmission guide. The durability of a particular laser energy soft tissue aspiration device is substantially related to the durability of the laser energy transmission guide. Particularly, laser energy aspiration devices must often be replaced or serviced when the tip or distal end of the laser energy transmission guide becomes charred or otherwise damaged. Devices according to the present invention can prevent such damage. For example, as described above, the laser energy transmission guide tip, e.g. a fiber tip, can be isolated from the aspiration lumen. Additionally, some embodiments locate the tip in a position such that it is outside of the flow of aspirated soft tissue. In some embodiments, the terminal point of the laser energy transmission guide is positioned distally relative, at least, to the proximal end of the aspiration inlet port and configured to direct laser energy within the lumen (e.g. via the reflection provided by an optical delivery system such as those described above). Further, in some embodiments, the terminal point of the laser energy transmission guide can be further removed from the flow of aspirated soft tissue by locating the terminal point at least at the mid-point of the aspiration inlet port(s), i.e. further from the aspiration inlet port's proximal end than the distal end. Further, in other embodiments, the terminal point of the laser energy transmission guide can be further removed from the flow of aspirated soft tissue by locating the terminal point at least three-fourths of the distance past the proximal end of the aspiration inlet port(s). Further still, some embodiments may completely remove the laser energy transmission guide terminal point from the flow of aspirated soft tissue by positioning said terminal point distally relative to the distal end of the aspiration inlet port(s). For example, the embodiment shown in Figure 5 includes such an arrangement with the fiber tip 508 positioned distally relative to the distal end of the aspiration inlet port 20.

For the above described embodiments, where appropriate the cannula, handle, laser guide tube, cannula tip, tip assembly outer tube, and tip assembly tip are all preferably of stainless steel. The cannula cross-sectional diameter can be between 1 mm and 8 mm, e.g. approximately 4 mm. For example in some embodiments, the cannula can comprise tubing of appropriate sizes such as: 0.312" Outer Diameter (O.D.) having a 0.016" wall (0.280" Inner Diameter); 0.250" O.D. having a 0.016" wall (0.218" I.D.); 0.188" O.D. having a 0.016" wall (0.156" I.D.); or 0.156" O.D. having a 0.016" wall (0.124" I.D.) all of variable length. As will be apparent to those of skill in this art, a shorter and thinner diameter aspiration cannula will be useful in more restricted areas of the body, as around small appendages, and a longer and larger diameter cannula will be useful in areas, such as the thighs and buttocks, where the cannula may be extended into fatty tissue over a more extensive area. The tip assembly outer tube is in sizes slightly larger than the cannula outer diameter and, in embodiments having an external laser guide tube, is still larger and possibly oblong shaped so as to fit around both the cannula and laser guide tube. The tip assembly tip 118 can be sized to a diameter slightly smaller than the outer tube so as to fit within the tube.

In another aspect of the invention, a device for in vivo, soft tissue lipolysis is disclosed. Embodiments of the device include a rigid laser energy transmission guide for insertion into a patient. In this aspect, the device can be subcutaneously inserted into a patient, and laser energy can be dispersed from the distal tip of the laser energy transmission guide. Laser energy at appropriate wavelengths and power levels, liquefies targeted soft tissue, and can simultaneously cauterize small veins and arteries at the lipolysis site. The liquefied tissue can be left at the site for absorption by lymphatic drainage, or can be subsequently removed by known tissue aspiration methods.

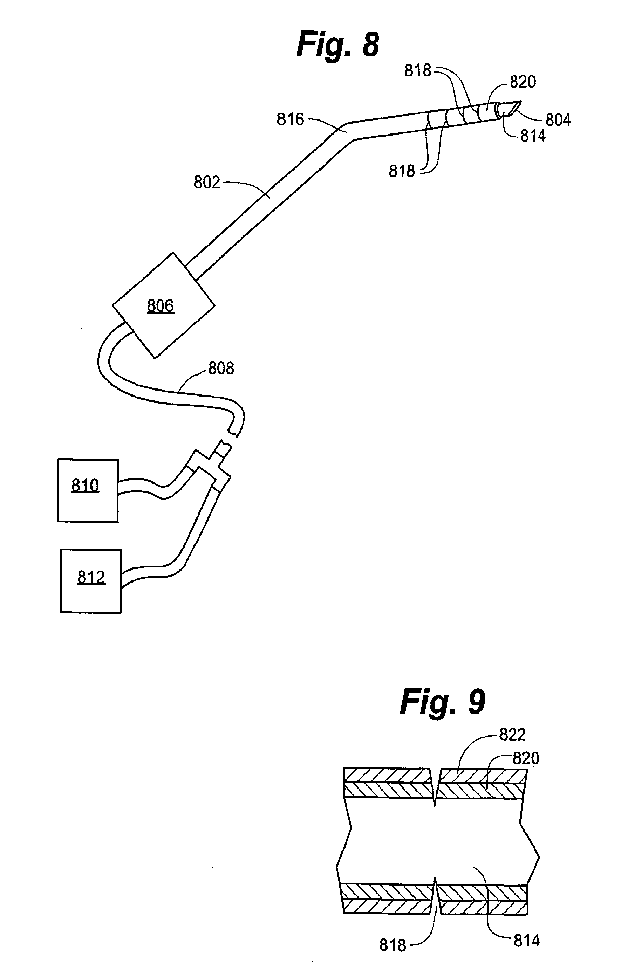

As shown in Figure 8, embodiments of the device include a rigid laser energy transmission guide 802 having a working distal tip 804. The rigid laser energy transmission guide 802 is optically coupled at junction 806 to an optical guide 808 coupled to a laser energy source 810 and an optional visible light source 812. The laser energy source 810 provides laser energy to the device for lipolysis of soft tissue. In a preferred embodiment, the laser energy source provides laser energy having a wavelength of 800 - 1200 nm and more preferrably 900-1100 nm (e.g. 976 nm or 1064 nm) at an adjustable power level ranging from 0-25 Watts. Optional visible light source 812 can provide light energy in the visible spectrum to allow an operator to follow (by transcutaneous vision) the position of the distal tip 804 within the patient's body. The laser energy source 810 and visible light source 812 can be any number of devices available on the market, and may comprise a single device. For example, in some embodiments the laser energy source can be an air-cooled diode laser source operating at 976 nm available from DILAS Diode Laser, Inc.

In many embodiments, the laser energy transmission guide 802 is a rigid optical fiber 814. Such a fiber can be constructed of an optically clear vitreous material such as quartz or silica glass. The rigid laser energy transmission guide 802 can be generally straight, or can include one or more shaping elements 816 such as, for example, a bend or curve at a desired location along the length of the device. Desired shaping elements can depend upon the location of the targeted tissue removal site within the patient.

The working distal tip 804 can be cleaved, molded, beveled, or otherwise formed to optimally disperse laser energy and maneuver within body tissue. As is commonly known in the field, during operation, optical fibers and guides often become charred at the distal end, decreasing energy distribution accuracy and efficiency. Thus, embodiments of the rigid laser energy transmission guide can be cleavable at the working distal tip 804. Some embodiments include a plurality of pre-cleave grooves 818, i.e. grooves within the coating of the fiber, slightly impinging on the cladding layer to allow for easier cleaving of the fiber tip during use, upon charring. The grooves 818 should be spaced lengthwise so as to allow for adequate removal of charred material, and should not be so deep as to threaten the structural integrity and optical transmission properties of the device. In a preferred embodiment, grooves are spaced 1 cm apart lengthwise, and penetrate the cladding of a 500 micron diameter fiber at a depth of no greater than 50 microns. Moreover, pre-cleave grooves 818 need not and in preferred embodiments, should not encircle the entire circumference of the rigid laser energy transmission guide 802. Rather, the pre-cleave grooves 818 can encircle only a portion, for example a 10 degree segment, of the circumference.

As can be seen in the section view of Figure 9, some embodiments can include a coating 820 encasing the fiber 814 along the fiber length. A coating can be used to enhance the optical and mechanical properties of the fiber, for example, the fiber 814 can include a silicone coating. In other embodiments, the fiber 814 may include a Teflon coating 820. Coating 820 may include pre-cleave grooves at predetermined locations (e.g. 2 mm to 2 cm lengths; in some embodiments 1 cm lengths) along the fiber length for easy, and accurate stripping. In some embodiments, the device further includes a tubing layer 822, surrounding the Teflon coating for increased strength, stiffness, and torque properties. For example, in one embodiment a tubing layer of Polymide - USP Class VI tubing available from Small Parts, Inc. 15901 SW 29^{th} St., Miramar, FL 33027 surrounds the Teflon jacket. Tubing layer 822 can likewise be pre-cleaved.

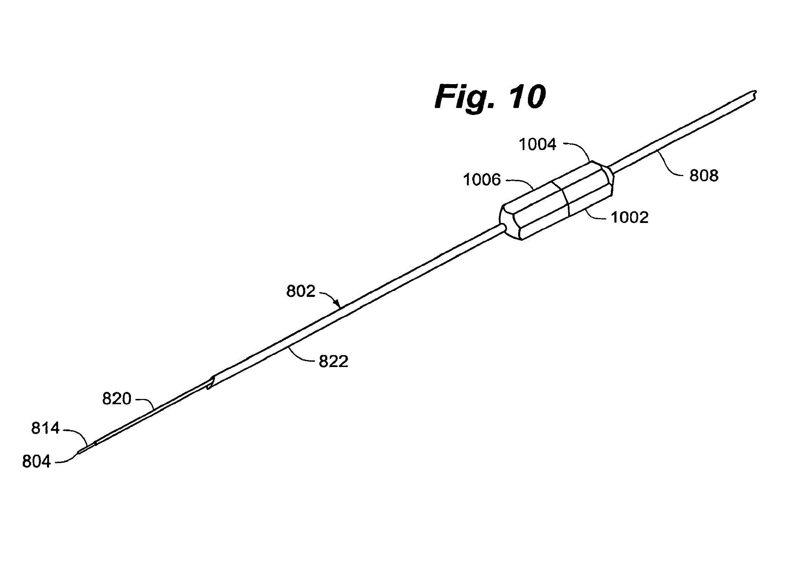

Junction 806 can be proximally located on the rigid laser energy transmission guide to provide an optical connection to an optical guide 808 coupled with a laser energy source 810 and optional visible light source 812. In some embodiments, for example that of Figure 10, the junction includes a collet 1002 or handle. Collet can have a first portion 1004 removably connectable to a second portion 1006 by threaded-, snap-, or other connection. For example, the interior of first portion 1004 can include a female threaded connector adapted to receive a male threaded connector on the interior of second portion 1006. When coupled together, first and second portions 1004, 1006 can frictionally engage rigid laser energy transmission guide 802 and optical guide 808 in optical communication with each other. In this manner, collet 1002 can provide for connection of the laser energy transmission guide 802 to a laser energy source. In some embodiments, the collet can be a hard plastic, ceramic, or other material capable of being autoclaved. In other embodiments, the collet can be disposable. In addition to coupling the rigid laser energy guide 802 with the optical guide 808, collet 1002 provides a grip or a handle allowing an operator to grasp the device and maneuver it to the lipolysis site. To this end, collet 1002 can include grips or other handle features to improve an operators handling of the device.

Also apparent in Figure 10 is stiffening tube 822 about the rigid laser energy transmission guide 802. Stiffening tube 822 can be a generally rigid, transparent tube having a beveled or flat end bonded to a cladding or sheath 820 of the fiber 814, or adhered directly to the fiber if no cladding layer is present. Stiffening tubes can further improve fiber rigidity along the length of the fiber and can be pared back and cut away like the tubing layers discussed above. In this view, the stiffening tube 822 has been stripped back from the distal end of the device to expose other features present. In some embodiments, the support tube 822 comprises polyamide.

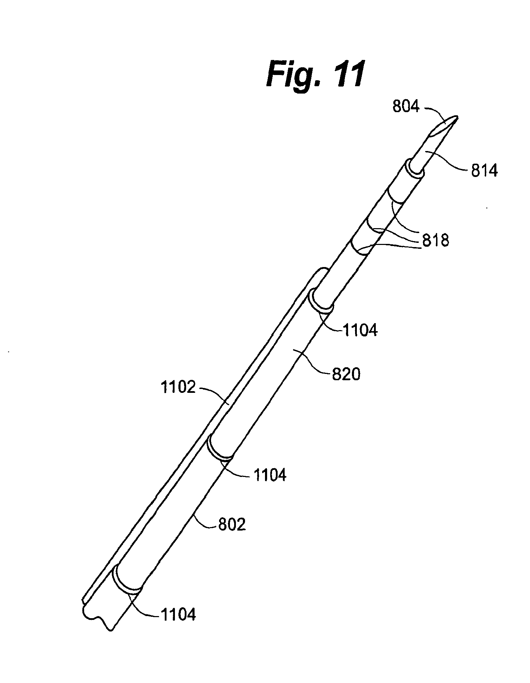

Figure 11 shows another embodiment of a rigid laser energy transmission guide 802. In this embodiment, rigid fiber 802 has been strengthened by the inclusion of a spine member 1102 longitudinally supporting the fiber along a portion of its length. Spine member 1102 can be coupled to rigid fiber 802 by a variety of mechanisms. In this embodiment, spine member 1102 includes a plurality of eyelets 1104 through which the rigid laser energy transmission guide 802 can pass. Working distal tip 804 and a portion of the rigid laser energy transmission guide 802 extend beyond the spine member 1102 and can include grooves 818 as described above. Spine member 1102 and eyelets 1104 should be constructed of a rigid material, such as for example, stainless steel.

To use an embodiment of a device including a rigid laser energy transmission guide, an operator can first make incision near the lipolysis site. The device can then be inserted, utilizing the rigidity of the laser energy transmission guide and any shaping elements present to guide the working distal tip to the lipolysis site. The operator can then activate the laser energy source to ablate and/or remove soft tissue and cauterize blood vessels at the lipolysis site. Depending upon the particular procedure, liquefied soft tissue can be left at the lipolysis site to be removed by the body, or may be suctioned out by insertion of a cannula or other device. If the fiber tip becomes charred during use, the fiber can be removed from the site, the working distal tip can be cleaved back to the sheath, and a portion of the sheath/cladding and tubing layer can be stripped (e.g. back to the next pre-cleaved groove if present). Lipolysis can then be resumed. Upon completion of the procedure, the device may be separated from the optical guide and disposed of, or in some cases, the fiber may be cleaved and autoclaved for future use.

In the foregoing detailed description, the invention has been described with reference to specific embodiments. However, it may be appreciated that various modifications and changes can be made without departing from the scope of the invention.

## Claims

1. An optical delivery system for a laser soft tissue aspiration device (100), comprising:
a laser energy transmission guide (115) adapted to deliver laser energy (228) from a terminal point of the laser energy transmission guide (115);
**characterized by**;
a lens (222, 504), in optical communication with the laser energy transmission guide (115) and adapted to isolate the terminal point from a lumen (113) of the laser soft tissue aspiration device (100); and
a reflective surface (224), in optical communication with the lens (222, 504) and adapted to reflect delivered laser energy (228) across an aspiration inlet port (20) of the laser soft tissue aspiration device (100),
wherein the lens (222, 504) is disposed distally relative to the aspiration inlet port (20) and the reflective surface (224) is disposed distally relative to the lens (222, 504).

2. The optical delivery system of claim 1,
further comprising a tip assembly (200) sized to fit over an open cannula (112) end of the laser soft tissue aspiration device (100), the lens (222, 504) and the reflective surface (224) being installed within the tip assembly (200).

3. The optical delivery system of claim 1,
wherein the lens (222, 504) and the reflective surface (224) are installed within a cannula (1 12) of the laser soft tissue aspiration device (100).

4. The optical delivery system of claim 1,
further comprising a window (220) abutting the lens.

5. The optical delivery system of claim 4,
further comprising a hole (226) in the window (220), the hole (226) adapted to receive the laser energy transmission guide (115).

6. The optical delivery system of claim 4,
further comprising an index matching gel disposed within a junction (518) between the window (502) and the lens (222, 504).

7. The optical delivery system of claim 1,
further comprising an optical epoxy layer for securing the lens (222, 504) relative to the aspiration inlet port (20).

8. The optical delivery system of claim 1,
wherein the lens (222, 504) is a collimating lens.

9. The optical delivery system of claim 1,
wherein the lens (222, 504) is a focusing lens.

## Patentansprüche

1. Optisches Ausgabesystem für eine Laser-Weichgewebeaspirationsvorrichtung (100), aufweisend:
einen Laserenergie-Übertragungsleiter (115), der dafür ausgelegt ist, Laserenergie (228) von einem Endpunkt des Laserenergie-Übertragungsleiters (115) auszugeben; **gekennzeichnet durch**
eine Linse (222, 504), die mit dem Laserenergie-Übertragungsleiter (115) in optischer Verbindung steht und dafür ausgelegt, den Endpunkt gegenüber einem Lumen (113) der Laser-Weichgewebeaspirationsvorrichtung (100) zu isolieren; und
eine reflektierende Oberfläche (224), die mit der Linse (222, 504) in optischer Verbindung steht und dafür ausgelegt ist, Laserenergie (228) über eine Aspirationseinlassöffnung (20) der Laser-Weichgewebeaspirationsvorrichtung (100) zu reflektieren,
wobei die Linse (222, 504) distal in Bezug auf die Aspirationseinlassöffnung (20) angeordnet ist, und die reflektierende Oberfläche (224) distal in Bezug auf die Linse (222, 504) angeordnet ist.

2. Optisches Ausgabesystem nach Anspruch 1,
ferner eine Spitzenanordnung (200) aufweisend, die so bemessen ist, dass sie über ein offenes Ende einer Kanüle (112) der Laser-Weichgewebeaspirationsvorrichtung (100) passt, wobei die Linse (222, 504) und die reflektierende Oberfläche (224) innerhalb der Spitzenanordnung (200) installiert sind.

3. Optisches Ausgabesystem nach Anspruch 1,
wobei die Linse (222, 504) und die reflektierende Oberfläche (224) innerhalb einer Kanüle (112) der Laser-Weichgewebeaspirationsvorrichtung (100) installiert sind.

4. Optisches Ausgabesystem nach Anspruch 1,
ferner ein Fenster (220) aufweisend, das an die Linse angrenzt.

5. Optisches Ausgabesystem nach Anspruch 4,
ferner ein Loch (226) in dem Fenster (220) aufweisend, wobei das Loch (226) dafür ausgelegt ist, den Laserenergie-Übertragungsleiter (115) aufzunehmen.

6. Optisches Ausgabesystem nach Anspruch 4,
ferner ein indexanpassendes Gel aufweisend, das in einem Übergangsbereich (518) zwischen dem Fenster (502) und der Linse (222, 504) angeordnet ist.

7. Optisches Ausgabesystem nach Anspruch 1,
ferner eine optische Epoxidschicht zum Befestigen der Linse (222, 504) in Bezug auf die Aspirationseinlassöffnung (20) aufweisend.

8. Optisches Ausgabesystem nach Anspruch 1,
wobei die Linse (222, 504) eine kollimatische Linse ist.

9. Optisches Ausgabesystem nach Anspruch 1,
wobei die Linse (222, 504) eine fokussierende Linse ist.

## Revendications

1. Système d'administration optique destiné à un dispositif laser d'aspiration de tissus mous (100), comprenant :
un guide de transmission d'énergie laser (115) adapté pour administrer de l'énergie laser (228) à partir d'un point terminal du guide de transmission d'énergie laser (115) ; **caractérisé par** ;
une lentille (222, 504) en communication optique avec le guide de transmission d'énergie laser (115) et adaptée pour isoler le point terminal d'une lumière (113) du dispositif laser d'aspiration de tissus mous (100) ;
et
une surface réfléchissante (224) en communication optique avec la lentille (222, 504) et adaptée pour réfléchir l'énergie laser administrée (228) à travers un orifice d'admission de l'aspiration (20) du dispositif laser d'aspiration de tissus mous (100), dans lequel la lentille (222, 504) est disposée distalement relativement à l'orifice d'admission de l'aspiration (20) et la surface réfléchissante (224) est disposée distalement relativement à la lentille (222, 504).

2. Système d'administration optique selon la revendication 1,
comprenant en outre un ensemble de pointes (200) dimensionné pour tenir au-dessus d'une extrémité de canule ouverte (112) du dispositif laser d'aspiration de tissus mous (100), la lentille (222, 504) et la surface réfléchissante (224) étant installées à l'intérieur de l'ensemble de pointes (200).

3. Système d'administration optique de la revendication 1,
dans lequel la lentille (222, 504) et la surface réfléchissante (224) sont installées à l'intérieur d'une canule (112) du dispositif laser d'aspiration de tissus mous (100).

4. Système d'administration optique selon la revendication 1.
comprenant en outre une fenêtre (220) en butée avec la lentille.

5. Système d'administration optique selon la revendication 4,
comprenant en outre un évidement (226) dans la fenêtre (220), l'évidement (226) étant adapté pour recevoir le guide de transmission de l'énergie laser (115).

6. Système d'administration optique selon la revendication 4,
comprenant en outre un gel adaptateur d'indice disposé à l'intérieur de la jonction (518) entre la fenêtre (502) et la lentille (222, 504).

7. Système d'administration optique selon la revendication 1,
comprenant en outre une couche optique d'époxy pour solidariser la lentille (222, 504) relativement à l'orifice d'admission de l'aspiration (20).

8. Système d'administration optique selon la revendication 1,
dans lequel la lentille (222, 504) est une lentille de collimation.

9. Système d'administration optique selon la revendication 1,
dans lequel les lentille (222, 504) est une lentille de focalisation.
